## Europäisches Patentamt
## European Patent Office
## Office européen des brevets

(11) Publication number: **0 033 659**
**A2**

# EUROPEAN PATENT APPLICATION

(21) Application number: **81300442.1**

(22) Date of filing: **03.02.81**

(51) Int. Cl.³: **A 61 M 25/00**

(30) Priority: **04.02.80 US 118411**

(43) Date of publication of application:
**12.08.81 Bulletin 81/32**

(84) Designated Contracting States:
**AT BE CH DE FR GB IT LI LU NL SE**

(71) Applicant: **TELEFLEX INCORPORATED**
**155 S. Limerick Road**
**Limerick Pennsylvania 19468(US)**

(72) Inventor: **Coneys, Thomas A.**
**6 Thackery Lane**
**Mendham New Jersey 07945(US)**

(74) Representative: **Wisher, Michael Frederick et al,**
**Urquhart-Dykes & Lord 47 Marylebone Lane**
**London W1M 6DL(GB)**

(54) Medical-surgical catheter.

(57) A medical-surgical catheter comprising an extruded tube of flexible material including a plastic material transparent to X-ray radiation and defining the entire interior and exterior surfaces of the tube to provide smooth surfaces of low coefficient of friction and an integrally extruded radiopaque layer completely embedded within and surrounded by the plastic material and extending along the tube between the ends thereof. The radiopaque layer comprises a blended mixture of radiopaque material and the plastic material with the blended mixture of the layer completely surrounded by a pure composition of the plastic material. The radiopaque layer may comprise an annular tube with an outer annular layer of the plastic material and an inner annular layer of the plastic material or the radiopaque layer may comprise a pair of diametrically opposed strips or the radiopaque layer may comprise a plurality of circumferentially spaced strips extending in a helical path along the tube.

Fig. 1

Croydon Printing Company Ltd.

EP 0 033 659 A2

# BACKGROUND OF THE INVENTION <span>0033659</span>

## (1) Field of the Invention

The subject invention relates to a medical-surgical tube such as that used in a catheter. The term "catheter" as used herein means any medical-surgical tube which includes a proximal end, a distal end and a longitudinal bore extending between the ends with the distal end having an opening. The catheter tubes are designed for insertion into some tissue, organ, cavity as, for example, a vascular or arterial branch, or the like, in a patient to serve as a channel for removing fluids from or introducing fluids into the tissue, organ, cavity, or the like, within the patient. Such catheters are frequently combined with syringes, valves, fluid traps or other units in creating assemblies required for the particular medical or clinical procedure being applied to the patient.

## (2) Description of the Prior Art

Extensive use is being made of disposable catheters designed for single patient and single use. Such disposable catheters are most commonly produced by an extrusion of waterproof and inert plastic material which is nontoxic, nonabsorbitive and resistant to attack or deterioration by fluids of body tissue into which the catheter may be inserted in the course of a surgical or clinical procedure. In many surgical or clinical procedures, it is important to be able to determine the location or position of the catheter within the body of the patient into which it has been inserted. Furthermore,

- 1 -

in some situations a tube inserted into the body of a patient may break off and it is imperative that a broken-off piece of tube be locatable. X-ray observation is a convenient method of making this position determination, but the usual plastic material from which catheters are made is not X-ray opaque, i.e., radiopaque. Accordingly, several catheter constructions have been devised in order to permit the body position of the catheter to be determined by X-ray observation. Such constructions include a radiopaque material combined or mixed with the plastic material of the catheter. However, the radiopaque material is frequently chemically reactive with body tissues and/or is rough or coarse and when exposed on the exterior of the catheter provides a relatively high coefficient of friction which causes irritation or impairs the insertion of the catheter into a tissue and when exposed to the interior of the tube provides a rough surface which frequently causes bruising of the blood which may, in turn, cause clotting. In some constructions the amount of radiopaque material is not sufficient for good detection of the placement of the catheter in all locations. If the amount of radiopaque material is increased for detection deep within the body, it may be unacceptable because of its roughness and/or because of it being too reactive. If the radiopaque material is sufficient for adequate radiopacity, the surface roughness and chemical reactivity is a problem and if reduced there may not be sufficient radiopacity.

## SUMMARY OF THE INVENTION    0033659

The subject invention provides a medical-surgical tube comprising an extruded tube of flexible material including a plastic material transparent to X-ray radiation and defining the entire interior and exterior surfaces of the tube to provide smooth surfaces of a low coefficient of friction and a radiopaque layer completely embedded within and surrounded by the plastic material and extending along the tube between the ends thereof so as to provide an improved catheter overcoming many of the disadvantages of the prior art catheters.

## PRIOR ART STATEMENT

An early construction of a catheter which is radiopaque is illustrated in United States Patent 2,212,334, granted August 20, 1940 to George W. Wallerich, wherein the catheter is made by first mixing bismuth powder with an appropriate cellulosic material and extruding the plastic compound. The radiopaque or X-ray opaque material is rough or coarse and is exposed to the inner and outer surfaces of the tube whereby the outer surface may be irritable to the skin tissues when inserted and the inner surfaces may cause bruising of the blood.

Another construction is shown in United States Patent 2,857,915, granted October 28, 1958 to David S. Sheridan, wherein a thin strip of radiopaque material is disposed in the outer circumference of the plastic tube. Again, the roughness of the radiopaque material of the strip is exposed to the outer surface of the catheter and, further, the amount of radiopaque material

- 3 -

in the strip is not sufficient for many X-ray purposes. A further example of a catheter utilizing a thin strip of opaque material exposed to the outer surface of the catheter is illustrated in United States Patent 3,295,527, granted January 3, 1967 to Ralph D. Alley et al.

United States Patent 3,529,633 granted September 22, 1970 to Vincent L. Vaillancourt, like United States Patent 2,212,334 discussed above, discloses a catheter with a portion of the circumference including plastic filled with a radiopaque material but differs from the aforementioned patent in that the remainder of the circumference of the tube consists of unfilled plastic. Again, however, the portion of the circumference of the tube having plastic filled with the radiopaque material has a relatively rough surface.

In another construction illustrated in United States Patent 3,605,750, granted September 20, 1971 to David S. Sheridan et al, there is disclosed a plastic tube with a bore extending longitudinally into the tube with short filaments of radiopaque material inserted into the bores. This construction provides a tube with a completely different structural integrity than the subject invention. Further, it is very difficult to place the radiopaque filaments in the bore and it is difficult to form the bore in the tube for receiving the spaced filaments.

In United States Patent 3,618,614, granted November 9, 1971 to Vincent J. Flynn, there is disclosed a catheter construction wherein the radiopaque material

- 4 -

P-528

0033659

is in a concentric annular tube either on the interior or the exterior of the plastic tube. The roughness of the radiopaque material either exteriorly or interiorly of the catheter is undesirable.

## BRIEF DESCRIPTION OF THE DRAWINGS

Other advantages of the present invention will be readily appreciated as the same becomes better understood by reference to the following detailed description when considered in connection with the accompanying drawings wherein:

FIGURE 1 is a view partially broken away and in cross section of the distal end of a medical-surgical catheter constructed in accordance with the subject invention;

FIGURE 2 is an enlarged cross-sectional view taken substantially along line 2-2 of FIGURE 1;

FIGURE 3 is a cross-sectional view similar to FIGURE 2 but showing an alternative embodiment; and

FIGURE 4 is a fragmentary perspective view partially broken away and in cross section showing yet another embodiment.

## DESCRIPTION OF THE PREFERRED EMBODIMENT

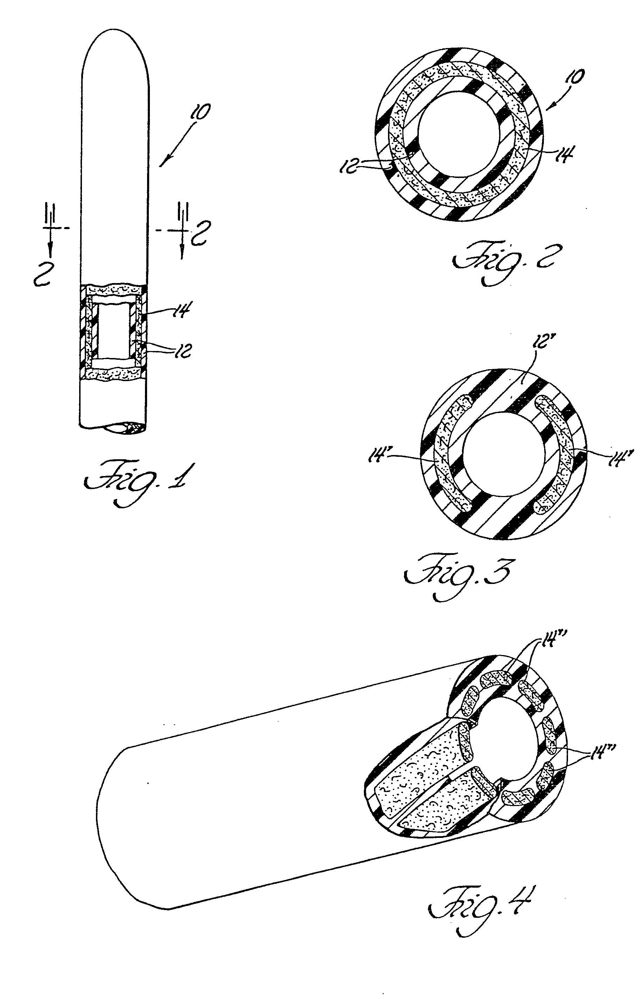

A medical-surgical catheter constructed in accordance with the subject invention is generally shown at 10 in FIGURES 1 and 2. The catheter includes an extruded tube of flexible material. The flexible material includes a plastic material transparent to X-ray radiation and defining the entire exterior and interior surfaces of the tube to provide smooth surfaces of a low

- 5 -

P-528

0033659

coefficient of friction. In other words, the plastic material 12 defines the inner tubular bore surface as well as the exterior surface of the tube. Further, the flexible material includes an integrally extruded radiopaque layer 14 completely embedded within and surrounded by the plastic material 12 and extending along the tube between the ends thereof. The end of the tube illustrated is usually sharp and exposes the central bore through an opening (not shown) for the passage of fluid. The plastic material 12 is preferably polyfluorinated ethylene-propylene but may be one of various alternative thermoplastic materials.

The radiopaque layer 14 comprises a blended mixture of radiopaque material and a binder material. The binder material and the plastic material are compatible for bonding and are bonded together. The binder material may be the same plastic material as the virgin or pure plastic material 12. In other words, the radiopaque layer 14 is a homogeneous mixture of a radiopaque material and the same kind of plastic as the pure plastic 12. The blended mixture of the layer 14 is completely surrounded by a pure composition of the plastic material 12. The radiopaque material is more coarse and abrasive than the plastic material defining the portions 12. The radiopaque material may be barium sulfate, bismuth trioxide, bismuth subcarbonate or tungsten powder or one of various other alternative radiopaque materials. By way of example, the blended mixture of the radiopaque material defining the layer 14 may include between twenty

- 6 -

percent (20%) and thirty percent (30%) fluorinated ethylene-propylene with the remaining seventy percent (70%) to eighty percent (80%) being one of the radiopaque materials identified above. The blended mixture is extruded simultaneously with the pure composition of the fluorinated ethylene-propylene 12 to define the catheter. Further, the radiopaque material preferably comprises between twelve percent (12%) and twenty-five percent (25%) of the total weight of the material making up the tube. In other words, the amount of radiopaque material in the blend making up the layer 14 comprises between twelve percent (12%) and twenty-five percent (25%) of the total material making up the tube. The radiopaque layer 14 comprises an annular tube with an outer annular layer of the plastic material thereabout to define the exterior surface and an inner annular layer of plastic material therewithin to define the interior surface. In other words, as viewed in cross section the radiopaque layer 14 is angular and extends completely about the circumference of the tube.

### DESCRIPTION OF THE ALTERNATIVE EMBODIMENTS

FIGURE 3 illustrates an alternative embodiment of the catheter of the subject invention wherein the pure plastic material 12' completely encapsulates and surrounds a pair of diametrically opposed strips 14' which define the radiopaque layer. The radiopaque layer defined by the strips 14'—also comprises a blend of radiopaque material and a plastic material the same as the plastic material 12'. Each of the strips 14' when viewed in cross section are arcuate to extend about a portion of the circumference of the tubular member so that the remaining portion of the circumference of the tube is made up solely of the plastic material 12' which, as in all embodiments, may be transparent whereby flow through the tube may be viewed visually.

Yet another embodiment is illustrated in FIGURE 4 wherein the radiopaque layer is made up of a plurality of strips 14'' spaced from one another circumferentially about the tube and extending in a helical path about the tube as the strips extend along the tube. The strips 14'' are also made up of a blend of the radiopaque material and the plastic of the type in which the strips 14'' are completely embedded and surrounded.

In accordance with the subject invention the radiopaque layer (the blend of the pure plastic and the radiopaque material) is completely embedded and surrounded on all surfaces by the pure composition of the plastic material thereby providing a catheter having surfaces with a low coefficient of friction yet providing a catheter having a very high concentration of the radiopaque material and chemical inertness which has been unattainable in the prior constructions.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that

0033659

within the scope of the appended claims, the invention may be practiced otherwise than as specifically described.

0033659

The embodiments of the invention in which an exclusive property or privilege is claimed are defined as follows:

1. A medical-surgical tube comprising; an extruded tube of flexible material including a plastic material transparent to X-ray radiation and defining the entire interior and exterior surfaces of the tube to provide smooth surfaces of a low coefficient of friction and a radiopaque layer completely embedded within and surrounded by said plastic material and extending along said tube between the ends thereof.

2. A tube as set forth in Claim 1 wherein said radiopaque layer comprises a blended mixture of radiopaque material and a binder material with the blended mixture of said layer completely surrounded by said plastic material.

3. A tube as set forth in Claim 2 wherein said binder material and said plastic material are compatible for bonding together.

4. A tube as set forth in Claim 3 wherein said binder material is the same material as said plastic material.

5. A tube as set forth in Claim 4 wherein said plastic material consists of polyfluorinated ethylene-propylene.

6. A tube as set forth in Claim 3 wherein said radiopaque material is more coarse and abrasive than said plastic material.

7. A tube as set forth in Claim 3 wherein said

P-528    CO33659

radiopaque layer comprises an annular tube with an outer annular layer of said plastic material thereabout to define said exterior surface and an inner annular layer of said plastic material therewithin to define said interior surface.

8.   A tube as set forth in Claim 3 wherein said radiopaque layer comprises at least one strip extending along said tube.

9.   A tube as set forth in Claim 7 including a pair of said strips diametrically opposed from one another, said strips extending annularly about a portion of the circumference of said tube with the remaining portion of the circumference consisting solely of said plastic material.

10.   A tube as set forth in Claim 8 including a plurality of said strips spaced from one another circumferentially about said tube and extending in a helical path about said tube as said strips extend along said tube.

11.   A medical-surgical tube comprising; an extruded tube of flexible material including a plastic material transparent to X-ray radiation and defining the entire interior and exterior surfaces of the tube to provide smooth surfaces of a low coefficient of friction and an integrally extruded radiopaque layer completely embedded within and bonded to and surrounded by said plastic material and extending along said tube between the ends thereof.

*Fig. 1*

*Fig. 2*

*Fig. 3*

*Fig. 4*